(19) Europäisches Patentamt
European Patent Office
Office européen des brevets

(11) Veröffentlichungsnummer: **0 172 407**
**B1**

(12) # EUROPÄISCHE PATENTSCHRIFT

(45) Veröffentlichungstag der Patentschrift: **23.01.91**

(51) Int. Cl.$^5$: **C 07 D 233/90**

(21) Anmeldenummer: **85108896.3**

(22) Anmeldetag: **16.07.85**

(54) **Verfahren zur Herstellung von Imidazol-4(5)-monocarbonsäuren, ihren Salzen oder Betainen.**

(30) Priorität: **24.07.84 DE 3427136**

(43) Veröffentlichungstag der Anmeldung:
**26.02.86 Patentblatt 86/09**

(45) Bekanntmachung des Hinweises auf die Patenterteilung:
**23.01.91 Patentblatt 91/04**

(84) Benannte Vertragsstaaten:
**BE CH DE FR GB IT LI NL**

(56) Entgegenhaltungen:
**DE-B-1 033 667**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Kempe, Uwe, Dr.**
**Danziger Strasse 9**
**D-6701 Dannstadt-Schauernheim (DE)**
Erfinder: **Dockner, Toni, Dr.**
**Grossgasse 6**
**D-6701 Meckenheim (DE)**
Erfinder: **Schlick, Rolf**
**Wielandstrasse 13**
**D-6700 Ludwigshafen (DE)**

**EP 0 172 407 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein neues Verfahren zur Herstellung von Imidazol-4(5)-monocarbonsäuren, ihren Salzen oder Betainen durch Umsetzung von Imidazolen mit Kohlendioxid und mit Alkalicarbonaten, Alkalihydrogencarbonaten und/oder Alkalihydroxiden bei einer Temperatur von 140 bis 230°C und einem Druck von 10 bis 350 bar und gegebenenfalls anschließender Umsetzung mit Säure.

Zur Herstellung von Imidazol-4(5)-monocarbonsäuren haben 2 Synthesewege praktische Bedeutung gewonnen: 1) die Oxidation von 4-Hydroxymethylimidazolen mit Salpetersäure (F. L. Pymann, J. Chem. Soc. *109,* 186—202 (1916), W. Hubball und F. L. Pymann, J. Chem. Soc. *131,* 21—32 (1928), R. Weidenhagen und H. Wegner, Ber. 70, 2309 (1937)) und 2) die partielle Decarboxilierung von 4,5-Imidazoldicarbonsäure oder 2-substituierten 4,5-Imidazoldicarbonsäuren in siedendem Anilin (R. G. Fargher und F. L. Pyman, J. Chem. Soc., *115,* 217 (1919) mit anschließender Hydrolyse der so gebildeten 4-Imidazolcarboxanilide durch Erhitzen in Säuren oder Laugen (CA 90: 72 189 z). Einen Weiteren Zugang zu Imidazol-4-carbonsäuren öffnet die oxidative Entschwefelung von 2-Mercaptoimidazol-4-carbonsäurederivaten (Weidenhagen, loc. cit. Seite 2310) sowie die Oxidation der entsprechenden Imidazoldithiocarbonsäuren mit Hydroperoxid (CA 98; 17 93 84 b (1983)). Allen diesen Verfahren ist gemeinsam, daß sie sich großtechnisch nur schwer durchführen lassen oder von teuren und großtechnisch schwierig durchführbaren Vorstufen ausgehen.

Die DE—B—1 033 667 beschreibt ein Verfahren zur Herstellung heterocyclischer Carbonsäuren durch Umsetzung von aromatischen Heterocyclen mit Kohlendioxid in Gegenwart von Alkalicarbonaten unter Druck. Als Temperaturbereich wird zwar ein Bereich oberhalb 150°C angegeben (Spalte 1, Zeile 8), jedoch darauf hingewiesen, daß bei verschiedenen Reaktionstemperaturen aus den gleichen Ausgangsstoffen verschiedene Produkte gebildet werden. So wird am Beispiel des Pyrazols (Spalte 1, Zeilen 15 bis 50) gezeigt, daß unter sonst gleichen Bedingungen bei 230°C das Salz der 3,5-Dicarbonsäure und bei höherer Temperatur, nämlich 270°C, das Salz der 4-Monocarbonsäure entsteht. Im Falle der Umsetzung von Imidazol und seinen Derivaten (Beispiele 1, 2, 5, 6, 11, 12 und 15) wird stets eine Temperatur von 250 bis 280°C verwendet; man erhält als Endstoffe stets Salze der entsprechenden Imidazol-4,5-dicarbonsäuren. Die beste Ausbeute an Imidazoldicarbonsäuren erhält man bei 260°C und einer Verwendung von Katalysatoren, nämlich Cadmiumfluorid (Beispiel 1). Wie die Beispiele zeigen, wird keine wesentliche Bildung von Imidazolmonocarbonsäuren festgestellt. Monocarbonsäuren erhält man, wie die Auslegeschrift zeigt, im Falle instabiler freier Dicarbonsäuren, z.B. von Triazolen-(1,2,4)-dicarbonsäuren, unter $CO_2$-Abspaltung (Spalte 2, Zeilen 20 bis 23). Die Beispiele zeigen, daß bei anderen Heterocyclen Monocarbonsäuren nur unter bestimmten Bedingungen entstehen, z.B. bei höherer Temperatur (260°C) und besonderer Struktur wie im Falle von Indazolen (Beispiel 13). Im Falle von Triazol und seiner 3-Monocarbonsäure wird bei 220°C (Beispiel 17) und höherer Temperatur, z.B. 260 bis 270°C (Beispiele 9, 16 und 19) die Dicarbonsäure erhalten, eine weitere Behandlung mit konzentrierter Salzsäure in der Hitze ist nötig, um ein Monocarbonsäuren bzw. Dicarbonsäure im wesentlichen Maße enthaltendes Reaktionsprodukt zu erzielen. Wie Beispiele und Beschreibung zeigen, werden die Ausbeuten der Reaktion und insbesondere die Reinheit des Endprodukts durch Katalysatoren begünstigt (Spalte 3, Zeilen 22 bis 37).

Es wurde nun gefunden, daß man Imidazol-4(5)-monocarbonsäuren der Formel (I)

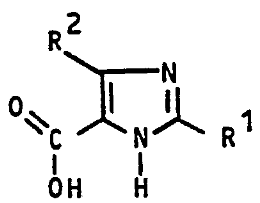

I

ihren Salzen oder Betainen, worin die einzelnen Reste $R^1$ und $R^2$ gleich oder verschieden sein können und jeweils ein Wasserstoffatom, einen Alkylkrest mit 1 bis 8 Kohlenstoffatomen, einen Cycloalkylrest mit 5 bis 8 Kohlenstoffatomen, einen Aralkyl- oder Alkylarylrest mit 7 bis 12 Kohlenstoffatomen oder einen Phenylorder Naphthylrest bedeuten, wobei die genannten Reste noch durch Alkyl- oder Alkoxygruppen mit je 1 bis 4 Kohlenstoffatomen substituiert sein können, mit der Maßgabe, daß mindestens einer der Reste $R^1$ oder $R^2$ für Wasserstoff steht, durch Umsetzung von Imidazolen mit Kohlendioxid und Alkaliverbindungen bei erhöhter Temperatur und unter Druck, vortailhaft erhält, wenn man Imidazole der Formel (II)

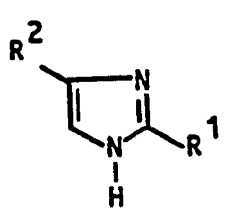

II

worin $R^1$ und $R^2$ die vorgenannte Bedeutung besitzen, mit 1 bis 100 Mol Kohlendioxid und mit 1 bis 4 Mol Alkalicarbonaten, Alkalihydrogencarbonaten und/oder Alkalihydroxiden je Mol Imidazol II bei einer

Temperatur von 140 bis 230°C und einem Druck von 10 bis 180 bar umsetzt und gegebenenfalls dann das Reaktionsprodukt mit Säure umsetzt.

Die Umsetzung läßt sich für den Fall der Verwendung von Kaliumcarbonat und 2,4-Dimethylimidazol durch die folgende Gleichung wiedergeben:

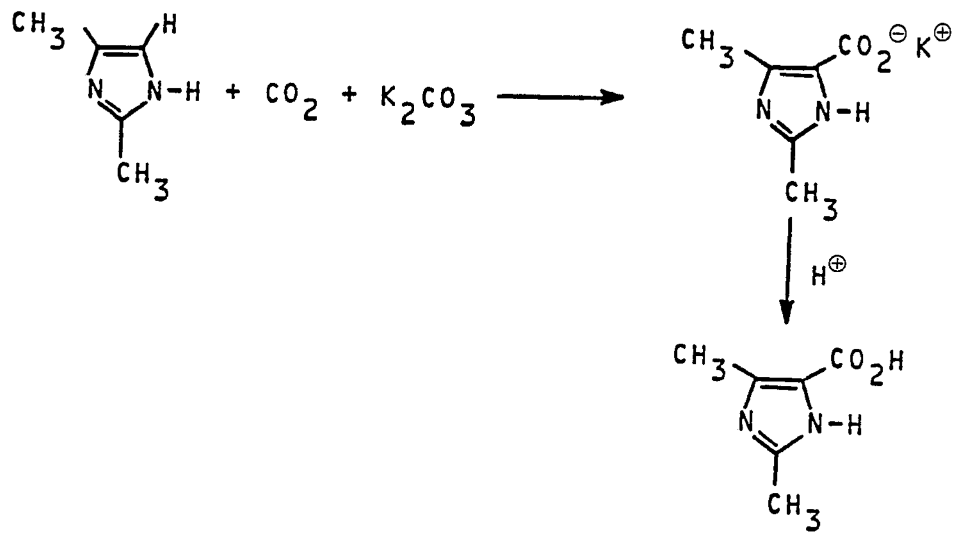

Im Vergleich mit dem bekannten Verfahren liefert das erfindungsgemäße Verfahren auf einfacherem und wirtschaftlicherem Wege Imidazol-4(5)-monocarbonsäuren und ihre Salze und Betaine in besserer Ausbeute und Reinheit. Alle diese vorteilhaften Ergebnisse sind im Hinblick auf den Stand der Technik überraschend. Man hätte mit Bezug auf die DE—B—1 033 667 erwartet, daß bei der tieferen erfindungsgemäßen Temperatur von höchstens 220°C nur die entsprechenden Dicarbonsäureverbindungen ohne wesentliche Mengen an Monocarbonsäure erhalten würden. Ebenfalls war es überraschend, daß der Endstoff in reiner Form und hoher Ausbeute anfällt, obgleich die Reaktionen in Abwesenheit von den in der DE—AS beschriebenen Metallkatalysatoren verläuft. Auch ist zur Herstellung der Monocarbonsäuren in den meisten Fällen überraschend keine Behandlung mit konzentrierter Salzsäure in der Hitze notwendig.

Die Umsetzung von Ausgangsstoff (II) mit Kohlendioxid kann in stöchiometrischer Menge oder im Überschuß jeder Komponenten zur anderen, zweckmäßig in einem Verhältnis von 1 bis 100, bevorzugt 5 bis 30 Mol Kohlendioxid je Mol Ausgangsstoff (II), durchgeführt werden. Kohlendioxid kann in fester, dampfförmiger oder zweckmäßig in flüssiger Form zugegeben werden.

Als Alkaliverbindungen werden Alkalihydroxide, Alkalihydrogencarbonate oder vorzugsweise Alkalicarbonate oder entsprechende Gemische verwendet. Bevorzugt kommen die Kaliumverbindungen in Frage. So sind z.B. die folgenden Alkaliverbindungen geeignet: Kaliumhydroxid, Kaliumhydrogencarbonat, bevorzugt Kaliumcarbonat. Die erfindungsgemäßen Alkaliverbindungen werden mit dem Ausgangsstoff (II) in stöchiometrischer Menge oder im Überschuß jeder Komponente zur anderen, vorteilhaft in einem Verhältnis von 1 bis 4 bevorzugt 1 bis 2,5 mol Alkaliverbindung je Mol Ausgangsstoff (II) umgesetzt.

Bevorzugte Ausgangsstoffe II und dem entsprechend bevorzugte Endstoffe I sind solche, in deren Formeln die einzelnen Reste $R^1$ und $R^2$ gleich oder verschieden sein können und jeweils ein Wasserstoffatom, einen Alkylrest oder einen mit Alkoxygruppen, insbesondere 1- und 2-Alkoxygruppen, zweckmäßig mit 1 bis 8, bevorzugt 1 bis 4 Kohlenstoffatomen je Alkoxygruppe, substituierten Alkylrest mit jeweils 1 bis 18, vorzugsweise 1 bis 8, insbesondere 1 bis 4 Kohlenstoffatomen, einen Aralkylrest oder Alkylarylrest mit 7 bis 12 Kohlenstoffatomen, einen Cycloalkylrest mit 5 bis 8 Kohlenstoffatomen oder einen Phenyl- oder Naphthylrest bedeuten mit der Maßgabe, daß mindestens einer der Reste $R^1$ oder $R^2$ für Wasserstoff steht. Die vorgenannten Reste können noch durch Alkyl- oder Alkoxygruppen mit 1 bis 4 Kohlenstoffatomen, substituiert sein.

Beispielsweise kommen folgende Imidazole als Ausgangsstoffe (II) in Frage: Methyl-, Ethyl-, Propyl-, i-Propyl-, Benzyl-, Phenyl-, Butyl-, sek.-Butyl-, iso-Butyl-, tert.-Butyl-, Cyclohexyl-imidazol und entsprechende 4(5)-Imidazole; Methoxy-, Ethoxy, Propoxy-, Isopropoxy-, Butoxy-, Isobutoxy-, sek-Butoxy-, tert.-Butoxymethyl-(2)-imidazol und entsprechende in der 1-Stellung der Alkylkette durch eine Methoxy-, Ethoxy-, Propoxy-, Isopropoxy-, Butoxy-, Isobutoxy-, sek.-Butoxy-, tert.-Butoxy-Gruppe substituierte Ethyl-, Propyl-, Isopropyl-, Butyl-, sek.-Butyl-, Isobutyl-(2)-imidazole; homologe, durch vorgenannte Alkoxyalkylgruppen in 4-Stellung am Ring substituierte Imidazole; Imidazol; bevorzugt sind Imidazol, 2-Methylimidazol, 2-Ethyl-, 2-Isopropyl-, 2-Phenyl-, 2-Cyclohexyl-, 2-Butylimidazol, 4-Methylimidazol, 2-Methoximethylimidazol.

Die Umsetzung wird bei einer Temperatur von 140 bis 230, zweckmäßig von 145 bis 220°C, bevorzugt 149 bis 220°C und einem Druck von 10 bis 180 bar, bevorzugt von 50 bis 160 bar kontinuierlich oder diskontinuierlich durchgeführt. Man kann zwar Katalysatoren verwenden, in der Regel wird aber in

Abwesentheit von Katalysatoren gearbeitet. Ebenfalls kann man auch in Gegenwart von organischen Lösungsmitteln, z.B. Methanol, Toluol, Dimethylformamid, umsetzen, jedoch wird im allgemeinen in Abwesenheit von organischen Lösungsmitteln bzw. in Abwesenheit von Wasser gearbeitet.

Die Reaktion kann wie folgt durchgeführt werden: Ein Gemisch von Ausgangsstoff (II), Kohlendioxid und Alkaliverbindung wird, zweckmäßig während 1 bis 30, vorteilhaft 1,5 bis 15 Stunden, bei der Reaktionstemperatur und dem Reaktionsdruck gehalten. Zweckmäßig verwendet man Ausgangsstoff (II) und Alkaliverbindung in feiner Verteilung.

Aus dem Reaktionsgemisch wird der Endstoff in üblicher Weise, z.B. durch Zerkleinerung des festen Reaktionsgemischs bzw. des festen Rückstands des Gemischs, Suspension in Wasser und Filtration, isoliert; der Endstoff liegt so im allgemeinen als wasserlösliches Salz der 4-Carbonsäure vor. Durch Ansäuern kann die freie 4-Carbonsäure aus dem Reaktionsgemisch isoliert werden. Zum Ansäuern können beliebige organische oder zweckmäßig anorganische Säuren, vorteilhaft Mineralsäuren, bevorzugt Salzsäure, Schwefelsäure und Phosphorsäure, verwendet werden. Bevorzugt wird beim Ansäuern der Suspension oder Lösung einen pH-Wert von 2 bis 6 eingestellt.

Im Endstoff liegt die Monocarbonsäure meist in Form seines Betains der Formel (III)

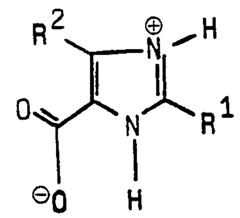

III

vor, worin $R^1$ und $R^2$ die vorgenannten allgemeinen und bevorzugten Bedeutungen besitzen.

Die nach dem Verfahren der Erfindung herstellbaren Imidazol-4(5)-monocarbonsäuren sind wertvolle Ausgangsstoffe für die Herstellung von Farbstoffen, Pflanzenschutzmitteln und Pharmazeutika. So sind Folgeprodukte der erfindungsgemäßen Endstoffe (I), nämlich die N-substituierten Monocarbonsäureester, z.B. der Ethylester, veterinärmedizinische Narkotika. Bezüglich der Verwendung wird auf die vergenannten Veröffentlichungen verwiesen.

### Beispiele 1 bis 7

Die in der Tabelle beschriebene Menge monosubstituiertes Imidazol, 0,3 Mol Kaliumcarbonat und 100 ml flüssiges Kohlendioxid wurden 5 Stunden in einem Autoklaven bei 150°C gerührt. Der Reaktionsdruck betrug 130 bar. Der Austrag wurde pulverisiert, in 100 ml Wasser suspendiert und unter Rühren mit Salzsäure auf einen pH von 5 bis 6 gestellt. Der ausfallende Niederschlag wurde abfiltriert und getrocknet.

Die Einsatzmengen und Ergebnisse sind in der folgenden Tabelle zusammengestellt:

| Beispiel | Ausgangsimidazol (II) $R^1$, $R^2$ | (g) | (Mol) | Imidazol-4-carbonsäure (I) $R^1$, $R^2$ | Ausbente (g) | (%) | Zersetsungs-punkt °C |
|---|---|---|---|---|---|---|---|
| 1 | 4-Methyl- | 16,4 | 0,2 | 5-Methyl- | 11,8 | 47 | 222 |
| 2 | 2-Methyl- | 16,4 | 0,2 | 2-Methyl- | 17,5 | 69 | 258 |
| 3 | 2-Ethyl- | 19,2 | 0,2 | 2-Ethyl- | 11,8 | 42 | 252 |
| 4 | 2-Isopropyl- | 11,0 | 0,1 | 2-Isopropyl- | 6,9 | 45 | 253 |
| 5 | 2-Phenyl- | 14,4 | 0,1 | 2-Phenyl- | 7,5 | 40 | 200 |
| 6 | 2-Methoxyimethyl- | 5,6 | 0,05 | 2-Methoximethyl- | 3,5 | 44,8 | 220 |
| 7 | 2-Benzyl | 15,8 | 0,1 | 2-Benzyl | 12 | 59 | 243 |

### Beispiel 8

544 g (8 Mol) Imidazol, 1380 g (10 Mol) Kaliumcarbonat und 1000 ml flüssiges $CO_2$ wurden 2,5 Stunden bei 120 bar und 219°C im Autoklaven zur Reaktion gebracht. Durch Ansäuern des Gemischs auf pH 3 und langsames (1 Stunde) ERwärmen auf 100°C wurde der Endstoff ausgefällt. Nach dem Trocknen erhielt man 606 g Imidazol-4-carbonsäure, die bei 220°C unter Zersetzung ($CO_2$-Entwicklung) schmolz. Dies entrspricht einer Ausbeute von 67,6% der Theorie.

### Beispiel 9

Die Umsetzung von Beispiel 8 wurde mit 10 Mol Kaliumhydroxid anstelle von Kaliumcarbonat

durchgeführt. Man erhielt Imidazol-4-carbonsäure in einer Ausbeute von 510 g (58% der Theorie) und einem Fp. 220°C.

**Patentansprüche**

1. Verfahren zur Herstellung von Imidazol-4(5)-monocarbonsäuren der Formel (I)

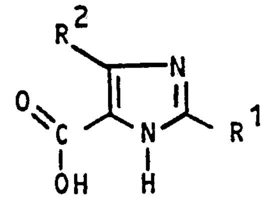

I

ihren Salzen oder Betainen, worin die einzelnen Reste $R^1$ und $R^2$ gleich oder verschieden sein können und jeweils ein Wasserstoffatom, einen Alkylkrest mit 1 bis 8 Kohlenstoffatomen, einen Cycloalkylrest mit 5 bis 8 Kohlenstoffatomen, einen Aralkyl- oder Alkylarylrest mit 7 bis 12 Kohlenstoffatomen oder einen Phenyl- oder Naphthylrest bedeuten, wobei die genannten Reste noch durch Alkyl- oder Alkoxygruppen mit je 1 bis 4 Kohlenstoffatomen substituiert sein können, mit der Maßgabe, daß mindestens einer der Reste $R^1$ oder $R^2$ für Wasserstoff steht, durch Umsetzung von Imidazolen mit Kohlendioxid und Alkaliverbindungen bei erhöhter Temperatur und unter Druck, dadurch gekennzeichnet, daß man Imidazole der Formel (II)

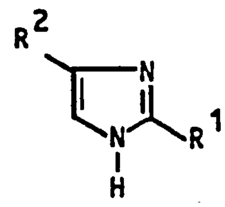

II

worin $R^1$ und $R^2$ die vorgenannte Bedeutung besitzen, mit 1 bis 100 Mol Kohlendioxid und mit 1 bis 4 Mol Alkalicarbonaten, Alkalihydrogencarbonaten und/oder Alkalihydroxiden je Mol Imidazol (II) bei einer Temperatur von 140 bis 230°C und einem Druck von 10 bis 180 bar umsetzt und gegebenenfalls dann das Reaktionsprodukt mit Säure umsetzt.

2. Verfahren nach Anspruch 1, dadurch gekennzeichnet, daß man 5 bis 30 Mol Kohlendioxid je Mol Ausgangsstoff (II) verwendet.

**Revendications**

1. Procédé de préparation d'acides imidazole-4(5)-monocarboxyliques de formule

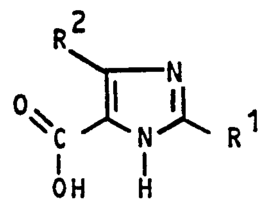

I

de leurs sels ou de leurs bétaînes, pour lesquels les symboles $R^1$ et $R^2$, qui peuvent avour des significations identiques ou différentes, représentent chacun un atome d'hydrogène, un groupe alkyle en C 1—C 8, un groupe cycloalkyle en C 5—C 8, un groupe aralkyle ou alkylaryle en C 7—C 12 ou un groupe phényle ou naphtyle, tous ces groupes pouvant encore être substitués per des groupes alkyle ou alcoxy contenant chacun 1 à 4 atomes de carbone, sous réserve que l'un au moins des symboles $R^1$ ou $R^2$ représente l'hydrogène, par réaction d'imidazoles avec le dioxyde de carbone et des composés alcalins à température élevée et sous pression, caractérisé en ce que l'on fait réagir des imidazoles répondant à la formule

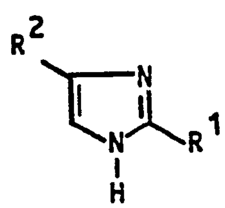

II

dans laquelle $R^1$ et $R^2$ ont les significations indiquées ci-dessus, avec 1 à 100 moles de dioxyde de carbone et avec 1 à 4 moles de carbonates alcalins, de bicarbonates alcalins et/ou d'hydroxydes alcalins par mile de l'imidazole II, à une température de 140 à 230 degrés C et une pression de 10 à 180 bar, et le cas échéant on fait réagir le produit obtenu avec un acide.

2. Procédé selon la revendication 1, caractérisé en ce que l'on utilise de 5 à 30 moles de dioxyde de carbone per mole du composant de départ II.

**Claims**

1. A process for the preparation of an imidazole-4(5)-monocarboxylic acid of the formula

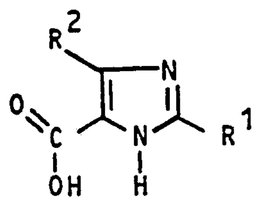

I

where $R^1$ and $R^2$ can be identical or different and are each hydrogen, alkyl of 1 to 8 carbon atoms, cycloalkyl of 5 to 8 carbon atoms, an aralkyl or alkylaryl radical of 7 to 12 carbon atoms or phenyl or naphthyl it being possible for the radicals mentioned to be further substituted by alkyl or alkoxy each of 1 to 4 carbon atoms, with the proviso that one or more of the radicals $R^1$ or $R^2$ are hydrogen, or its salts or betaines, by reacting an imidazole with carbon dioxide and an alkali metal compound at elevated temperatures and under superatmospheric pressure, wherein an imidazole of the formula

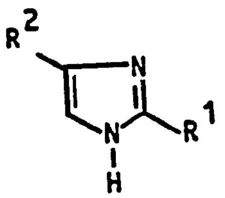

II

where $R^1$ and $R^2$ have the above meanings, is reacted with from 1 to 100 moles of carbon dioxide and from 1 to 4 moles of an alkali metal carbonate, an alkali metal bicarbonate and/or an alkali metal hydroxide per mole of imidazole II at from 140 to 230°C and under from 10 to 180 bar and, if desired, the product is then reacted with an acid.

2. A process as claimed in claim 1, wherein from 5 to 30 moles of carbon dioxide are used per mole of starting material II.